# EUROPÄISCHE PATENTSCHRIFT

(11) **EP 0 920 340 B1**
(45) Veröffentlichungstag und Bekanntmachung des Hinweises auf die Patenterteilung: **22.11.2000**
(21) Anmeldenummer: 97927109.5
(22) Anmeldetag: 03.06.1997
(51) Int. Cl.: A61L 2/18, C23C 14/18, G02C 13/00

(54) **VORRICHTUNG ZUR KATALYTISCHEN ZERSETZUNG VON WASSERSTOFFPEROXYD**
DEVICE FOR CATALYTICALLY DISSOLVING HYDROGEN PEROXIDE
DISPOSITIF DE DISSOLUTION CATALYTIQUE DE PEROXYDE D'HYDROGENE

(30) Priorität: 17.06.1996 DE 19624095
(43) Veröffentlichungstag der Anmeldung: 09.06.1999
(73) Patentinhaber: MDLE Medical Device Laboratories Europe GmbH, 87700 Memmingen (DE)
(72) Erfinder: MÜLLER-LIERHEIM, Wolfgang, G., K., D-81477 München (DE)
(74) Vertreter: Nöth, Heinz, Dipl.-Phys.
(86) Internationale Anmeldenummer: EP9702863
(87) Internationale Veröffentlichungsnummer: WO9748423

(56) Entgegenhaltungen:
- WO-A-93/06870
- DE-A- 3 843 746
- DE-A- 4 243 410
- DE-A- 19 522 950
- US-A- 5 275 784

## Beschreibung

Die Erfindung betrifft eine Vorrichtung nach dem Oberbegriff des Patentanspruches 1.

Bei einer derartigen aus der US 5,275,784 A bekannten Vorrichtung ist es zur Kontaktlinsenpflege bekannt, katalytisch wirkendes Platinmetall, beispielsweise durch Sputtern, auf einer Unterlage aus Polymermaterial zur Wasserstoffperoxydzersetzung in einer wäßrigen Pflegelösung als Schicht abzuscheiden. Schwierigkeiten bereitet hier die ausreichende Haftfestigkeit zwischen der katalytisch aktiven Platinschicht und der Kunststoffunterlage. Für die Abscheidung der katalytischen Platinschicht sind relativ große Platinmengen erforderlich.

Aus der deutschen Patentschrift DE 24 25 714 B2 ist es bekannt, Kontaktlinsen, insbesondere Weichkontaktlinsen, mittels Wassserstoffperoxyd in einer wäßrigen, insbesondere 3%igen wäßrigen Wasserstoffperoxydlösung zu sterilisieren. Zur Beseitigung von Resten des Wasserstoffperoxyds werden diese mit Hilfe eines Zersetzungskatalysators in Wasser und Sauerstoff zersetzt. Diese Zersetzung kann auch in dem Behandlungsbad zur Sterilisier- und Desinfektionsbehandlung der Kontaktlinse durchgeführt werden.

Der Katalysator besitzt ein katalytisch inaktives Trägermaterial, auf welchem eine katalytisch aktive Schicht aufgebracht ist. Diese Schicht ist beim bekannten Katalysator aus elektrolytisch abgelagertem Platinschwarz gebildet.

Aus der US 5,306,352 A ist es ebenfalls bekannt, nach der Sterilisierung von Kontaktlinsen mittels Wasserstoffperoxyd Reste des Wasserstoffperoxyds mittels eines geeignetes Katalysators zu zersetzen, wobei das Katalysatormaterial auf einem Träger oder Substrat aus Kunststoff aufgebracht wird.

Das nicht vorveröffentlichte Dokument DE-A-19 522 950 beschreibt eine Vorrichtung, die einen Aufnahmebehälter aufweist sowie einen Formkörper, der auf einer TiOₓN_{y}-Unterlage eine Platinschicht aufweist.

Aufgabe der Erfindung ist es, eine Vorrichtung der eingangs genannten Art zu schaffen, bei der eine verbesserte Haftfestigkeit zwischen Platinschicht und Unterlagen erreicht wird.

Diese Aufgabe wird erfindungsgemäß durch das Kennzeichen des Patentanspruches 1 gelöst.

Auf der bevorzugt aufgerauhten Oberfläche des Formkörpers aus Glas kann die Platinschicht als äußerst dünner Film aufgebracht werden, wobei dieser Film bevorzugt die Oberflächenrauhigkeit nachbildet. Auf diese Weise wird nicht nur eine verbesserte Haftfestigkeit zwischen dem katalytisch wirkenden Platinfilm und der Unterlage erreicht, sondern es wird eine verringerte Masse an Platinmetall bei der Schichtbildung benötigt. Man erreicht auf geringem Volumen des Formkörpers bzw. mit verringerter Fläche am Formkörper eine ausreichend große katalytisch aktive Oberflächenschicht am Formkörper. Eine bevorzugte Schichtdicke für die Platinschicht liegt im Bereich von 0,1 bis 1 g/m².

Die Oberflächenrauhigkeit wird bevorzugt durch eine mechanische Oberflächenbehandlung, insbesondere durch Partikelstrahlen, erreicht. In bevorzugter Weise kommt als Strahlverfahren ein Sandstrahlverfahren zur Anwendung bei der Aufrauhung der Oberfläche der Unterlage. Es hat sich herausgestellt, daß eine durch Sandstrahlen aufgerauhte Glasoberfläche eine hervorragende Hafteigenschaft gegenüber der Platinschicht, insbesondere, wenn diese durch Sputtern aufgebracht wird, aufweist. Durch Sputtern läßt sich eine extrem dünne Schicht auf der durch Sandstrahlen aufgerauhten Glasoberfläche erreichen.

Diese durch Sputtern erzeugte Platinschicht wird dadurch gewonnen, daß ein Platintarget mit energiereichen Ionen eines inerten Stoffes, beispielsweise Argon, Neon und dergl. beschossen wird, so daß Platin in reiner Form sich als Platinschicht auf dem Formkörper abscheidet. Die durch Sputtern abgeschiedene Platinschicht besitzt auf dem insbesondere durch Sandstrahlen aufgerauhten Formköprer eine hohe Haftfestigkeit und kann in gleichbleibender Dicke als dünner Film gebildet werden. Die gleichbleibende dünne Schicht mit hoher Reinheit läßt sich in ihrer Dicke reproduzierbar durch entsprechende Verfahrensführung beim Sputtering gesteuert aufbringen. Vor allem läßt sich die Platinschicht in der Weise bemessen, daß sie beim Einsatz des Katalysators im wäßrigen, insbesondere 3%igem Wasserstoffperoxydpflegesystem für Kontaktlinsen eingesetzt werden kann. Diese Schicht läßt sich so gestalten, daß eine ausreichende Sterilisierwirkung durch das Wasserstoffperoxyd auf die zu behandelnden Kontaktlinsen, insbesondere Weichkontaktlinsen, erreicht wird bei anschließender Zersetzung noch vorhandenen Restwasserstoffperoxyds in der Behandlungslösung.

Die katalytisch wirkende Vorrichtung kann bevorzugt Bestandteil eines Kontaktlinsenpflegesystems sein, bei welchem die Kontaktlinse in einem Behälter eingebracht wird und in diesem Behälter mit Hilfe wäßriger Pflegemittellösung mit 3% Wasserstoffperoxydgehalt die Pflege und Sterilisierung der Kontaktlinse durchgeführt wird. Die Platinschicht ist in der Weise eingestellt, daß eine ausreichende Einwirkungszeit des Wasserstoffperoxyds zur Desinfektion der zu behandelnden Kontaktlinsen im Behälter erreicht wird und daß anschließend an die Desinfektionsbehandlung restliches Wasserstoffperoxyd wirkungsvoll in die Bestandteile Wasser und Sauerstoff zersetzt wird.

Anhand der Figuren wird an Ausführungsbeispielen die Erfindung noch näher erläutert. Es zeigt:
- Fig. 1:: ein erstes Ausführungsbeispiel;
- Fig. 2:: ein zweites Ausführungsbeispiel; und
- Fig. 3:: schematisch einen Teil der katalytisch wirksamen Oberfläche bei den beiden Ausführungsbeispielen.

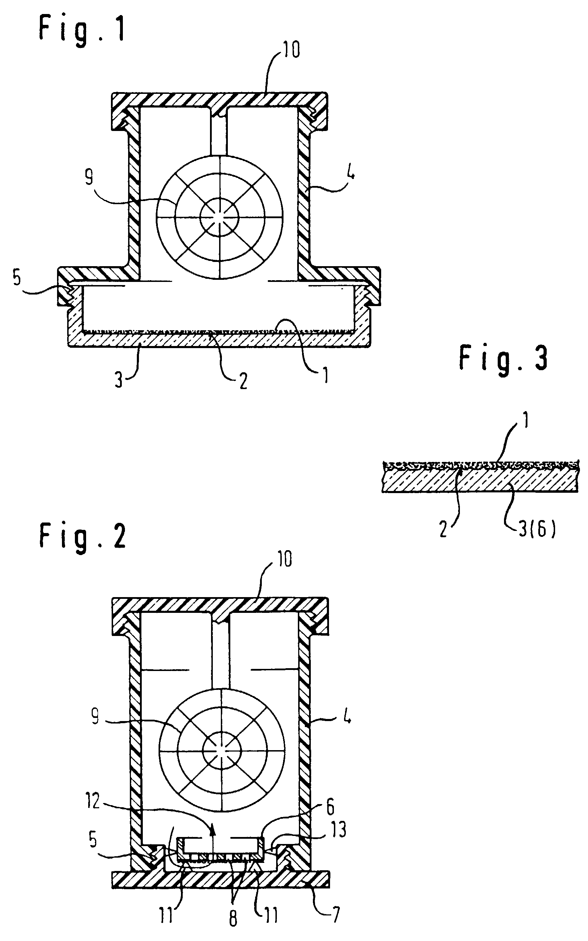

Die in den Figuren 1 und 2 dargestellten Ausführungsbeispiele werden angewendet bei der Kontaktlinsenpflege und zeigen jeweils einen Behälter 4, der als Aufbewahrungs- bzw. Pflegebehälter für Kontaktlinsen verwendet werden kann. Die zu behandelnden Kontaktlinsen befinden sich in einem aufklappbaren Korb 9, wie er beispielsweise aus der US 5,275,784 A bekannt ist. Bei der Pflege werden die zu behandelnden Kontaktlinsen in eine wäßrige Pflegemittellösung, die einen 3%igen Wasserstoffperoxydgehalt hat, im Behälter 4 eingetaucht. Der Korb 9 kann an einem auf den Behälter 4 aufschraubbaren Deckel 10 befestigt sein.

Zum Verschließen des Bodens besitzt das Ausführungsbeispiel der Fig. 1 ein Bodenteil 3, welches mittels Schraubgewindeeingriff 5 am unteren Teil des Behälters 4 aufschraubbar ist. Das Ausführungsbeispiel der Fig. 2 besitzt ebenfalls einen aufschraubbaren Behälterboden 7.

Bei dem in der Fig. 1 dargestellten Ausführungsbeispiel besteht der Behälterboden bzw. das aufschraubbare Bodenteil 3 bevorzugt aus Glas. Das Bodenteil 3 bildet den Formkörper bzw. die Unterlage für eine katalytisch wirkende Platinschicht 1, welche auf die Innenseite des Bodenteils 3 aufgebracht ist. Die Oberfläche, welche mit der Platinschicht 1 belegt wird, ist vorher durch ein mechanisches Behandlungsverfahren, insbesondere Strahlverfahren, bei welchem mit Partikelstrahlen, insbesondere Sandstrahlen, die Oberfläche behandelt wurde, aufgerauht worden, so daß eine aufgerauhte Oberfläche 2 auf dem Glasmaterial des Bodenteils 3 entsteht. Die Konfiguration der aufgerauhten Oberfläche 2 auf der Glasunterlage (Bodenteil 3) und der darauf aufgebrachten Platinschicht ist schematisch in der Fig. 3 dargestellt. Wie aus dieser Figur zu ersehen ist, paßt sich die Platinschicht 1, welche bevorzugt durch Sputtern aufgebracht wurde, den Rauhigkeiten der Oberfläche an und hat gegenüber der Glasunterlage eine erhöhte Haftfestigkeit aufgrund der Rauhigkeit der Oberfläche, welche bevorzugt durch Sandstrahlbehandlung erzeugt worden ist. Beim dargestellten Ausführungsbeispiel der Fig. 1 ist im wesentlichen der gesamte Bodenbereich, d. h. die innere Oberfläche des Bodenteils 3 aufgerauht und mit der katalytisch wirkenden Platinschicht belegt.

Bei dem in der Fig. 2 dargestellten Ausführungsbeispiel kann der aufschraubbare Behälterboden 7 aus dem gleichen Kunststoffmaterial bestehen wie der Behälter 4. Am Behälterboden 7 ist ein Einsatz 6 vorgesehen in Form einer gelochten oder Bohrungen 8 aufweisenden Glasplatte. Die Glasplatte ist bevorzugt an ihrer Unterseite gemäß Fig. 3 aufgerauht und mit der Platinschicht 1 versehen. Es kann jedoch auch die Oberseite aufgerauht sein und mit der Platinschicht 1 versehen sein. In gleicher Weise wird auch bei diesem Ausführungsbeispiel die Platinschicht 1 durch Sputtern aufgebracht. Die Unterseite der gelochten Glasplatte des Einsatzes 6 wird durch Abstandhalter 11 in einem bestimmten Abstand vom Behälterboden 7 gehalten. Hierdurch wird gewährleistet, daß der bei der katalytischen Zersetzung entstehnde Sauerstoff in Form von Sauerstofblasen aufsteigt und so eine Zirkulation verursacht, so daß zu zersetzendes Wasserstoffperoxyd zirkuliert (Pfeil 12) und wirkungsvoll zersetzt wird. Zur Unterstützung der Zirkulation können noch seitliche Abstandhalter 13 vorgesehen sein.

## Patentansprüche

1. Vorrichtung zur katalytischen Zersetzung von Wasserstoffperoxyd (H₂O₂) in einer wässrigen Lösung mit einem Aufnahmebehälter, in welchem die wässrige Lösung enthalten ist, einem mit Platin als Katalysator beschichteten Formkörper mit großer Oberfläche, wobei die Oberfläche der Platinschicht in unmittelbarer Berührung mit der das Wasserstoffperoxyd enthaltenden wässrigen Lösung steht,
dadurch **gekennzeichnet**,
daß der Formkörper aus Glas besteht.

2. Vorrichtung nach Anspruch 1, dadurch gekennzeichnet, daß der Formkörper (3;6) durch mechanische Oberflächenbehandlung aufgerauht ist, vorzugsweise durch Partikelstrahlen oder durch Sandstrahlen.

3. Vorrichtung nach einem der Ansprüche 1 bis 2, dadurch gekennzeichnet, daß die Platinschicht (1) durch "Sputtern" auf die aufgerauhte Oberfläche (2) des Formkörpers (3;6) aufgebracht ist.

4. Vorrichtung nach einem der Ansprüche 1 bis 3, dadurch gekennzeichnet, daß der Formkörper (3;6) an einem Aufbewahrungsbehälter (4) lösbar befestigt ist.

5. Vorrichtung nach Anspruch 4, dadurch gekennzeichnet, daß der Formkörper (3) ein Bodenteil des Aufbewahrungsbehälters (4) bildet.

6. Vorrichtung nach Anspruch 3 oder 4, dadurch gekennzeichnet, daß der Formkörper (3;6) mittels Schraubverbindung (5) am Aufbewahrungsbehälter (4) befestigt ist.

7. Vorrichtung nach einem der Ansprüche 1 bis 4, dadurch gekennzeichnet, daß der Formkörper (6) als Einsatz im Aufbewahrungsbehälter (4) ausgebildet ist.

8. Vorrichtung nach Anspruch 7, dadurch gekennzeichnet, daß der Einsatz (6) Bohrungen (8) aufweist, die in einem bestimmten Abstand vom Behälterboden (7) angeordnet sind.

9. Vorrichtung nach Anspruch 8, dadurch gekennzeichnet, daß die Bohrungen (8) in einem etwa parallel zum Behälterboden (7) verlaufenden Einsatzteil vorgesehen sind.

10. Vorrichtung nach einem der Ansprüche 1 bis 9, dadurch gekennzeichnet, daß die Platinschicht (1) in einem wäßrigen Kontaktlinsenpflegemittel angeordnet ist.

11. Vorrichtung nach Anspruch 10, dadurch gekennzeichnet, daß das Kontaktlinsenpflegemittel eine 3%-ige H₂O₂-Lösung zur Desinfektion von Kontaktlinsen ist.

## Claims

1. Apparatus for the catalytic decomposition of hydrogen peroxide (H₂O₂) in an aqueous solution comprising a receiving container in which the aqueous solution is contained, and a shaped body with a large surface area, which is coated with platinum as a catalyst, wherein the surface of the platinum layer is in direct contact with the hydrogen peroxide-bearing aqueous solution, characterised in that the shaped body comprises glass.

2. Apparatus according to claim 1 characterised in that the shaped body (3; 6) is roughened by mechanical surface treatment, preferably by particle blasting or by sand blasting.

3. Apparatus according to one of claims 1 and 2 characterised in that the platinum layer (1) is applied by sputtering to the roughened surface (2) of the shaped body (3; 6).

4. Apparatus according to one of claims 1 to 3 characterised in that the shaped body (3; 6) is releasably secured to a storage container (4).

5. Apparatus according to claim 4 characterised in that the shaped body (3) forms a bottom portion of the storage container (4).

6. Apparatus according to claim 3 or claim 4 characterised in that the shaped body (3; 6) is secured to the storage container (4) by means of a screw connection (5).

7. Apparatus according to one of claims 1 to 4 characterised in that the shaped body (6) is in the form of an insert in the storage container (4).

8. Apparatus according to claim 7 characterised in that the insert (6) has bores (8) which are arranged at a given spacing from the container bottom (7).

9. Apparatus according to claim 8 characterised in that the bores (8) are provided in an insert portion extending substantially parallel to the container bottom (7).

10. Apparatus according to one of claims 1 to 9 characterised in that the platinum layer (1) is arranged in an aqueous contact lens care agent.

11. Apparatus according to claim 10 characterised in that the contact lens care agent is a 3% H₂O₂ solution for disinfecting contact lenses.

## Revendications

1. Dispositif pour la décomposition catalytique de peroxyde d'hydrogène (H₂O₂) dans une solution aqueuse, comprenant un conteneur de réception dans lequel la solution aqueuse est contenue, un corps moulé revêtu de platine en tant que catalyseur, de grande surface, la surface de la couche de platine étant au contact direct de la solution aqueuse contenant le peroxyde d'hydrogène, caractérisé en ce que le corps moulé est constitué de verre.

2. Dispositif selon la revendication 1, caractérisé en ce que le corps moulé (3 ; 6) est rendu rugueux au moyen d'un traitement mécanique de la surface, de préférence au moyen de faisceaux de particules ou au moyen de jets de sable.

3. Procédé selon l'une des revendications 1 et 2, caractérisé en ce que la couche de platine (1) est appliquée par "pulvérisation" sur la surface (2) rendue rugueuse du corps moulé (3 ; 6).

4. Procédé selon l'une des revendications 1 à 3, caractérisé en ce que le corps moulé (3 ; 6) est fixé de façon détachable sur un conteneur de garde (4).

5. Dispositif selon la revendication 4, caractérisé en ce que le corps moulé (3) forme une partie de fond du conteneur de garde (4).

6. Dispositif selon la revendication 3 ou 4, caractérisé en ce que le corps moulé (3 ; 6) est fixé sur le conteneur de garde (4) au moyen d'un assemblage par vissage (5).

7. Dispositif selon l'une des revendications 1 à 3, caractérisé en ce que le corps moulé (6) a la configuration d'un insert dans le conteneur de garde (4).

8. Dispositif selon la revendication 7, caractérisé en ce que l'insert (6) présente des trous (8) qui sont disposés à une distance définie du fond (7) du conteneur.

9. Dispositif selon la revendication 8, caractérisé en ce que les trous (8) sont prévus dans une partie insérée orientée approximativement parallèlement au fond (7) du conteneur.

10. Dispositif selon l'une des revendications 1 à 9, caractérisé en ce que la couche de platine (1) est disposée dans un agent aqueux de nettoyage de lentilles de contact.

11. Dispositif selon la revendication 10, caractérisé en ce que l'agent de nettoyage de lentilles de contact est une solution de H₂O₂ à 3 % pour la désinfection de lentilles de contact.
